# EUROPEAN PATENT SPECIFICATION

(11) **EP 2 659 930 B1**
(45) Date of publication and mention of the grant of the patent: **30.08.2017**
(21) Application number: 13164420.5
(22) Date of filing: 19.04.2013
(51) Int. Cl.: A61N 1/05, A61N 1/36

(54) **Cochlear lead**
Cochlea-Leitung
Conducteur cochléaire

(30) Priority: 03.05.2012 US 201213463450
(43) Date of publication of application: 06.11.2013
(73) Proprietor: Advanced Bionics AG, 8712 Stäfa (CH)
(72) Inventor: Thenuwara, Chuladatta, Castaic, CA 91384 (US); Downing, Mark, Valencia, CA 91355 (US)
(74) Representative: Schwan Schorer & Partner mbB

(56) References cited:
- WO-A1-02/28473
- WO-A1-2011/149695
- US-A1- 2003 093 139
- US-A1- 2011 319 907
- US-A1- 2012 035 615
- US-B1- 6 421 569

## Description

The present invention relates to a cochlear lead.

Hearing loss can be corrected using a number of approaches, including surgically placing a cochlear implant which includes an electrode array that is inserted into the cochlea of a patient. The electrode array presents electrical stimulation directly to auditory nerve fibers in the cochlea. This leads to the perception of sound in the brain and provides at least partial restoration of hearing function.

WO 2011/149695 discloses a cochlear lead having the features of the preamble of claim 1 and relates to a cochlear lead comprising a plurality of electrodes configured to stimulate an auditory nerve from within the cochlea, a flexible body supporting the electrodes along a length of the flexible body, and a stiffening element slidably encapsulated within the flexible body and positioned such that the stiffening element plastically deforms upon insertion into a curved portion of the cochlea.

WO 02/28473 A1 relates to a cochlear lead comprising a plurality of electrodes supported by a flexible body and a stiffening element located within a lumen of the flexible body which is provided with slits allowing ingress of fluid into the lumen for allowing the stiffening element to dissolve or soften as a result of fluid ingress.

Occasionally, the cochlear implant may need to be replaced with a new cochlear implant. The original electrode array is removed from the cochlea and a new cochlear lead is inserted. In some instances, the cochlea may have tissue that at least partially occludes the passageway into which the new cochlear lead is to be inserted. This presents a number of challenges to be addressed by a new cochlear lead design particularly suitable for revision surgery.

According to the invention, this object is achieved by a cochlear lead as defined in claim 1. The invention is beneficial in that, with the stiffening element extending past the nost distal electrode, the cochlear lead is particularly suitable for insertion into an ossified cochlea in revision surgery.

Peferred embodiments are defined in the dependent claims.

The accompanying drawings illustrate various embodiments of the principles described herein and are a part of the specification. The illustrated embodiments are merely examples and do not limit the scope of the claims.
Fig. 1 is a diagram showing an illustrative cochlear implant system in use, according to one example of principles described herein.
Fig. 2 is a diagram showing external components of an illustrative cochlear implant system, according to one example of principles described herein.
Fig. 3 is a diagram showing the internal components of an illustrative cochlear implant system, according to one example of principles described herein.
Fig. 4 is a cross-sectional view of a cochlea with an illustrative cochlear lead which includes an electrode array and stiffening element, according to one example of principles described herein.
Fig. 5A is a partial side view of an illustrative stiffening element and sheath, according to one example of principles described herein.
Fig. 5B shows a cross-section of an illustrative stiffening element with a sheath in place and a cap placed over the distal end of the stiffening element, according to one example of principles described herein.
Fig. 5C shows a cross-section of a tip of an illustrative cochlear lead, according to one example of principles described herein.
Fig. 6A is a side view of an illustrative cochlear lead, according to one example of principles described herein.
Figs. 6B-6C are cross-sectional views of the cochlear lead shown in Fig. 6A, according to one example of principles described herein.
Figs. 7A-7C are cross sectional diagrams of steps in an illustrative process for making a cochlear lead with an integral stiffening element, according to one example of principles described herein.
Fig. 8 is a flowchart showing an illustrative method for forming a cochlear lead, according to one example of principles described herein.
Fig. 9 is a flowchart showing an illustrative method for using a cochlear lead, according to one example of principles described herein.

Throughout the drawings, identical reference numbers designate similar, but not necessarily identical, elements.

In human hearing, hair cells in the cochlea respond to sound waves and produce corresponding auditory nerve impulses. These nerve impulses are then conducted to the brain and perceived as sound.

Hearing loss, which may be due to many different causes, is generally of two types: conductive and sensorineural. Conductive hearing loss typically occurs where the normal mechanical pathways for sound to reach the hair cells in the cochlea are impeded, for example, from damage to the ossicles. Conductive hearing loss may often be helped by using conventional hearing aids that amplify sounds so that acoustic information can reach the cochlea and the hair cells. Some types of conductive hearing loss are also treatable by surgical procedures.

Many people who are profoundly deaf, however, have sensorineural hearing loss. This type of hearing loss can arise from the absence or the destruction of the hair cells in the cochlea which then no longer transduce acoustic signals into auditory nerve impulses. Individuals with sensorineural hearing loss may be unable to derive significant benefit from conventional hearing aid systems alone, no matter how loud the acoustic stimulus is. This is because the mechanism for transducing sound energy into auditory nerve impulses has been damaged. Thus, in the absence of properly functioning hair cells, auditory nerve impulses cannot be generated directly from sounds.

To overcome sensorineural deafness, cochlear implant systems, or cochlear prostheses, have been developed that can bypass the hair cells located in the cochlea by presenting electrical stimulation directly to the auditory nerve fibers. This leads to the perception of sound in the brain and provides at least partial restoration of hearing function. Most of these cochlear prosthesis systems treat sensorineural deficit by stimulating the ganglion cells in the cochlea directly using an implanted lead that has an electrode array. Thus, a cochlear prosthesis operates by directly stimulating the auditory nerve cells, bypassing the defective cochlear hair cells that normally transduce acoustic energy into electrical activity to the connected auditory nerve cells.

A cochlear implant system typically comprises both an external unit that receives and processes ambient sound waves and an implanted processor/cochlear lead that receives data from the external unit and uses that data to directly stimulate the auditory nerve. The cochlear lead includes an electrode array that is implanted within one of the cochlear ducts, such as the scala tympani. To minimize damage to sensitive tissues within the patient's cochlea, it can be desirable for the electrode array to be accurately placed within the cochlea using a minimum amount of insertion force. The cochlear implant should be designed so that the insertion forces do not kink or otherwise damage the delicate wires and electrodes contained within the implant.

According to one illustrative embodiment, the electrode array can be constructed from biocompatible silicone, platinum-iridium wires, and platinum electrodes. The portion of the lead to be inserted into the cochlea is designed to be relatively flexible so that it can curve around the helical interior of the cochlea.

This specification describes a stiffening element that provides a desired level of rigidity to the electrode array for improved control by the surgeon and prevents kinking along the length of the cochlear lead during insertion. In some embodiments, the stiffening element extends past the most distal electrode to the tip of the electrode array. This stiffening element can be particularly suited for revision surgeries. The stiffening element is more rigid than the body of the cochlear lead and is fully encapsulated within the lead to reduce the risk of infection and better stabilize it within the lead. According to one illustrative embodiment, this stiffening element serves at least four purposes. First, the stiffening element provides additional rigidity along the length of the cochlear lead, thereby reducing the likelihood that the insertion forces will kink the lead. Second, the stiffening element provides the surgeon with greater control over the placement of the lead within the cochlea. Third, the stiffening element redirects the insertion force into a tangential force, which allows the cochlear lead to be inserted deeper into the cochlea with less applied force. Fourth, at least a portion of the stiffening element is formed from a material that plastically deforms during insertion, which allows the stiffening element to conform to the shape of the cochlea and provides the ability to overcome obstructive tissue and provide full insertion of the electrode array.

Revision surgery can be used to explant a cochlear electrode array from the cochlea and replace it with a new electrode array. During revision surgery it is sometimes difficult to insert a compliant pre-curved or lateral electrode array due to the presence of scar tissue/ossification that has formed around the previous electrode array. For a partially ossified cochlea or for a cochlea with fibrous tissue growth, a stiffer electrode can be used to ensure insertion to the full depth of the electrode. In one example, a cochlear lead includes an integral stiffener with suitable malleability. The integral stiffener extends along the complete length of the electrode array and conforms to the shape of the cochlea.

In the following description, for purposes of explanation, numerous specific details are set forth in order to provide a thorough understanding of the present systems and methods. It will be apparent, however, to one skilled in the art that the present systems and methods may be practiced without these specific details. Reference in the specification to "an embodiment," "an example," or similar language means that a particular feature, structure, or characteristic described in connection with the embodiment or example is included in at least that one embodiment, but not necessarily in other embodiments. The various instances of the phrase "in one embodiment" or similar phrases in various places in the specification are not necessarily all referring to the same embodiment.

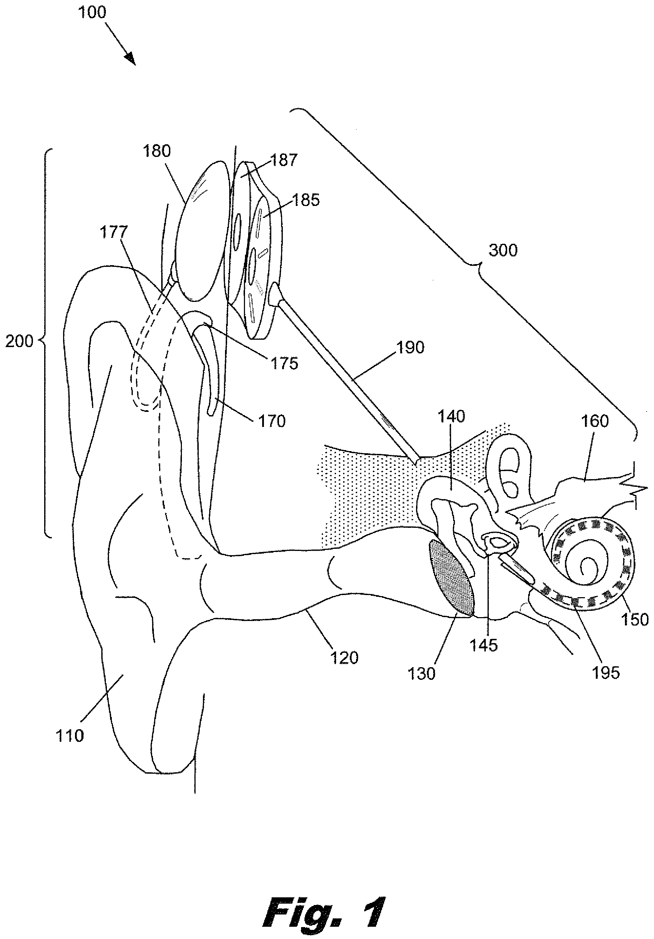

Fig. 1 is a diagram showing one illustrative embodiment of a cochlear implant system 100 having a cochlear implant 300 with an electrode array 195 that is surgically placed within the patient's cochlea. Ordinarily, sound enters the external ear, or pinna, 110 and is directed into the auditory canal 120 where the sound wave vibrates the tympanic membrane 130. The motion of the tympanic membrane is amplified and transmitted through the ossicular chain 140, which consists of three bones in the middle ear. The third bone of the ossicular chain 140, the stirrup 145, contacts the outer surface of the cochlea 150 and causes movement of the fluid within the cochlea. Cochlear hair cells respond to the fluid-borne vibration in the cochlea 150 and trigger neural electrical signals that are conducted from the cochlea to the auditory cortex by the auditory nerve 160.

As indicated above, the cochlear implant 300 is a surgically implanted electronic device that provides a sense of sound to a person who is profoundly deaf or severely hard of hearing. In many cases, deafness is caused by the absence or destruction of the hair cells in the cochlea, i.e., sensorineural hearing loss. In the absence of properly functioning hair cells, there is no way auditory nerve impulses can be directly generated from ambient sound. Thus, conventional hearing aids, which amplify external sound waves, provide no benefit to persons suffering from complete sensorineural hearing loss.

Unlike hearing aids, the cochlear implant 300 does not amplify sound, but works by directly stimulating any functioning auditory nerve cells inside the cochlea 150 with electrical impulses representing the ambient acoustic sound. Cochlear prosthesis typically involves the implantation of electrodes into the cochlea. The cochlear implant operates by direct electrical stimulation of the auditory nerve cells, bypassing the defective cochlear hair cells that normally transduce acoustic energy into electrical energy.

External components 200 of the cochlear implant system can include a Behind-The-Ear (BTE) unit 175, which contains the sound processor and has a microphone 170, a cable 177, and a transmitter 180. The microphone 170 picks up sound from the environment and converts it into electrical impulses. The sound processor within the BTE unit 175 selectively filters and manipulates the electrical impulses and sends the processed electrical signals through the cable 177 to the transmitter 180. The transmitter 180 receives the processed electrical signals from the processor and transmits them to the implanted antenna 187 by electromagnetic transmission. In some cochlear implant systems, the transmitter 180 is held in place by magnetic interaction with a magnet 189 in the underlying antenna 187.

The components of the cochlear implant 300 include an internal processor 185, an antenna 187, and a cochlear lead 190 having an electrode array 195. The internal processor 185 and antenna 187 are secured beneath the user's skin, typically above and behind the pinna 110. The antenna 187 receives signals and power from the transmitter 180. The internal processor 185 receives these signals and performs one or more operations on the signals to generate modified signals. These modified signals are then sent through the cochlear lead 190 to the electrode array 195, which is the portion of the cochlear lead 190 that is implanted within the cochlea 150 and provides electrical stimulation to the auditory nerve 160.

The cochlear implant 300 stimulates different portions of the cochlea 150 according to the frequencies detected by the microphone 170, just as a normal functioning ear would experience stimulation at different portions of the cochlea depending on the frequency of sound vibrating the liquid within the cochlea 150. This allows the brain to interpret the frequency of the sound as if the hair cells of the basilar membrane were functioning properly.

The cochlear lead typically comprises an electrode array that is implanted in the scala tympani. The electrode array typically includes several separately connected stimulating electrode contacts, conventionally numbering about 6 to 30, longitudinally disposed on a thin, elongated, flexible carrier. The electrode array is pushed into the scala tympani duct in the cochlea, typically to a depth of about 13 to 30 mm via a cochleostomy or via a surgical opening made in the round window at the basal end of the duct.

In use, the cochlear electrode array delivers electrical current into the fluids and tissues immediately surrounding the individual electrode contacts to create transient potential gradients that, if sufficiently strong, cause the nearby auditory nerve fibers to generate action potentials. The auditory nerve fibers branch from cell bodies located in the spiral ganglion, which lies in the modiolus, adjacent to the inside wall of the scala tympani. The density of electrical current flowing through volume conductors such as tissues and fluids tends to be highest near the electrode contact that is the source of such current. Consequently, stimulation at one contact site tends to selectively activate those spiral ganglion cells and their auditory nerve fibers that are closest to that contact site.

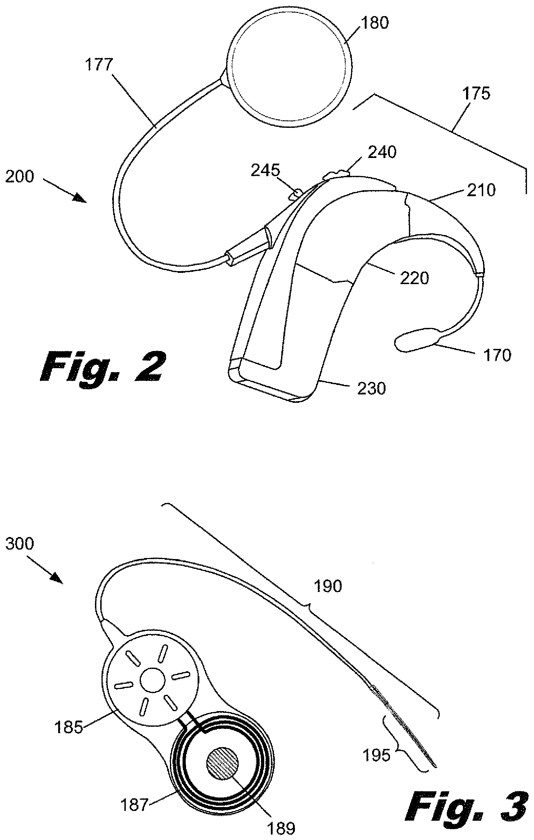

Fig. 2 is an illustrative diagram showing a more detailed view of the external components 200 of one embodiment of a cochlear implant system. External components 200 of the cochlear implant system include a BTE unit 175, which comprises a microphone 170, an ear hook 210, a sound processor 220, and a battery 230, which may be rechargeable. The microphone 170 picks up sound from the environment and converts it into electrical impulses. The sound processor 220 selectively filters and manipulates the electrical impulses and sends the processed electrical signals through a cable 177 to the transmitter 180. A number of controls 240, 245 adjust the operation of the processor 220. These controls may include a volume switch 240 and program selection switch 245. The transmitter 180 receives the processed electrical signals from the processor 220 and transmits these electrical signals and power from the battery 230 to the cochlear implant by electromagnetic transmission.

Fig. 3 is an illustrative diagram showing one embodiment of a cochlear implant 300, including an internal processor 185, an antenna 187, and a cochlear lead 190 having an electrode array 195. The cochlear implant 300 is surgically implanted such that the electrode array 195 is internal to the cochlea, as shown in Fig. 1. The internal processor 185 and antenna 187 are secured beneath the user's skin, typically above and behind the pinna 110, with the cochlear lead 190 connecting the internal processor 185 to the electrode array 195 within the cochlea. According to one illustrative embodiment, the electrode array 195 is straight or slightly curved before being inserted into the cochlea 150. As discussed below, this particular electrode array 195 is designed for revision surgery. However, the principles described can be applied to a broad range of medical devices. As discussed above, the antenna 187 receives signals from the transmitter 180 and sends the signals to the internal processor 185. The internal processor 185 modifies the signals and passes them through the cochlear lead 190 to the electrode array 195. The electrode array 195 is inserted into the cochlea and provides electrical stimulation to the auditory nerve. This provides the user with sensory input that is a representation of external sound waves sensed by the microphone 170.

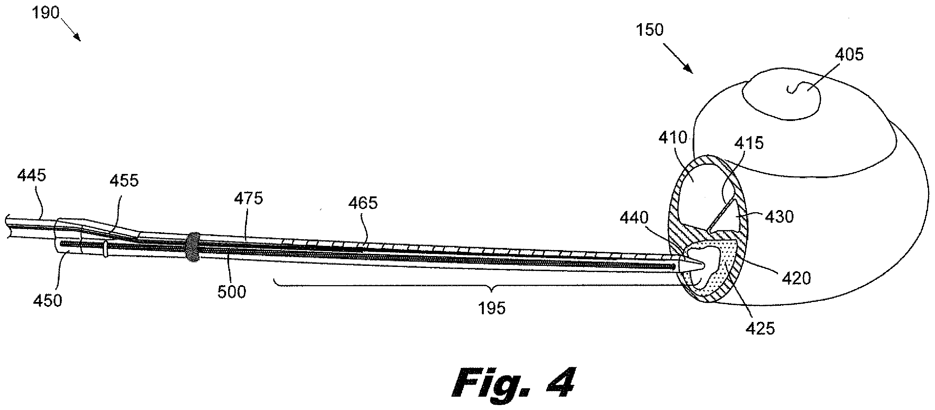

Fig. 4 is a partially cutaway perspective view of a cochlea 150 and shows an illustrative electrode array 195 being inserted into the cochlea 150. The primary structure of the cochlea is a hollow, helically coiled, tubular bone, similar to a nautilus shell. The coiled tube is divided through most of its length into three fluid-filled spaces (scalae). The scala vestibuli 410 is partitioned from the scala media 430 by Reissner's membrane 415 and lies superior to it. The scala tympani 420 is partitioned from the scala media 430 by the basilar membrane 425 and lies inferior to it. A typical human cochlea includes approximately two and a half helical turns of its constituent channels. The cochlear lead 190 is inserted into one of the scalae, typically the scala tympani 420, to bring the individual electrodes into close proximity with the tonotopically organized nerves.

Throughout the specification and appended claims the term "distal" refers to portions that are closer to the tip 440 of the cochlear lead 190 and "proximal" refers to portions that are farther away from the tip 440. The terms "medial" and "lateral" refer to locations that are closer to the center of the cochlea and closer to the outer portions of the cochlea, respectively. For example, the phrase medial wall of the cochlea refers to portions of a cochlear duct that are toward the center of the cochlea.

In the example shown in Fig. 4, the scala tympani 420 is narrowed by ossification 425. The ossification and other obstructions in the scala may form for a variety of reasons, including foreign body reaction to a previous electrode array. The ossification or other obstructions may partially block the scala and make the insertion of a new electrode array during revision surgery challenging. In particular, the surgeon may need increased control to maneuver the electrode array around or through the obstructions. Higher forces may be needed to successfully insert the electrode array to the desired insertion depth. The combination of higher forces and obstructions can lead to kinking or folding of the electrode array. In one embodiment, it can be desirable to insert the electrode array to 360 degrees or approximately the same depth as the previous electrode array.

The illustrative cochlear lead 190 is specifically designed to provide the surgeon with the desired control and prevent the electrode array from kinking or folding along its length. The cochlear lead 190 includes a lead body 445. The lead body 445 connects the electrode array 195 to the internal processor 185 (Fig. 3). A number of wires 455 pass through the lead body 445 to bring electrical signals from the internal processor 185 (Fig. 3) to the electrode array 195. According to one illustrative embodiment, proximal of the electrode array 195 is a molded silicone rubber feature 450. The feature 450 can serve a variety of functions, including, but not limited to, providing a structure that can be gripped or pushed by an insertion tool and providing a visual indicator of how far the cochlear lead 190 has been inserted.

The wires 455 that conduct the electrical signals generated by the processor are connected to the electrodes 465 within the electrode array 195. For example, electrical signals which correspond to a low frequency sound may be communicated via a first wire to an electrode near the tip 440 of the electrode array 195. Electrical signals which correspond to a high frequency sound may be communicated by a second wire to an electrode 465 near the proximal end of the electrode array 195. According to one illustrative embodiment, there may be one wire 455 for each electrode 465 within the electrode array 195. The internal processor 185 (Fig. 3) may then control the electrical field generated by each electrode individually. For example, one electrode may be designated as a ground electrode. The remainder of the electrodes may then generate electrical fields which correspond to various frequencies of sound. Additionally or alternatively, adjacent electrodes may be paired, with one electrode serving as a ground and the other electrode being actively driven to produce the desired electrical field.

According to one illustrative embodiment, the wires 455 and portions of the electrodes 465 are encased in a flexible body 475. The flexible body 475 may be formed from a variety of biocompatible materials, including, but not limited to, medical grade silicone rubber. The flexible body 475 secures and protects the wires 455 and electrodes 465. The flexible body 475 allows the electrode array 195 to bend and conform to the geometry of the cochlea. When placed within the cochlea 150, the electrode array 195 brings the individual electrodes into close proximity with the tonotopically organized nerves in the cochlea 150.

Additionally, as can be seen in Fig. 4, and described further below, a stiffening element 500 extends from the molded silicone rubber feature 450 to the tip 440 of the electrode array 195. The stiffening element 500 allows the cochlear lead to be more precisely positioned within the cochlea and reduces the propensity of the cochlear lead 190 to kink. In embodiments where the stiffening element 500 is plastically deformable, the stiffening element 500 conforms to the curvature of the cochlea during insertion. This can help prevent undesirable motion of the lead within the cochlea. The stiffening element and its function are described in more detail below.

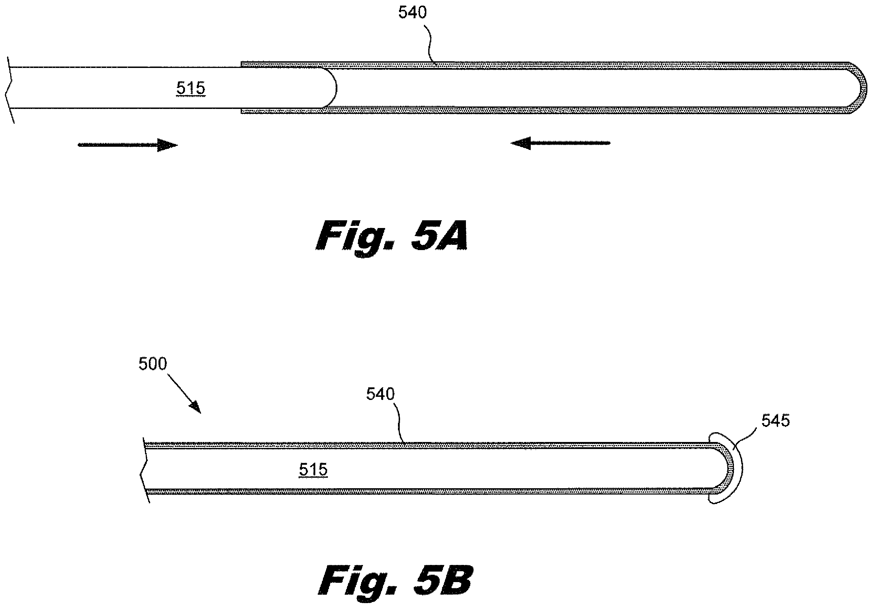

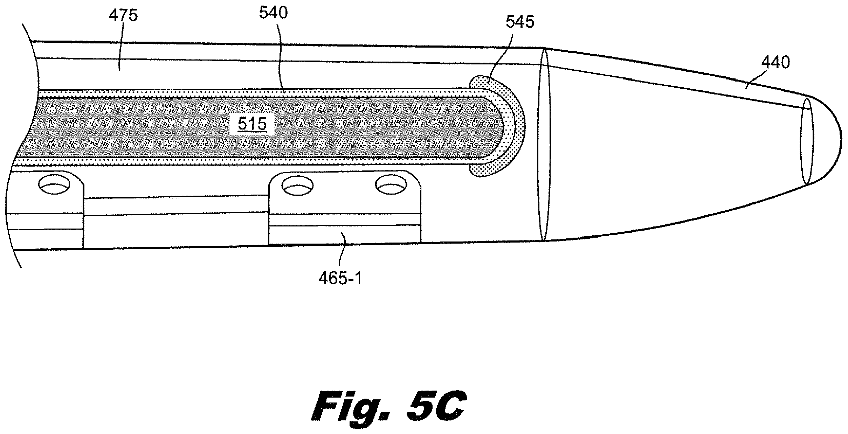

Figs. 5A and 5B are detail views of an illustrative stiffening element. Fig. 5A is a side view of an illustrative stiffener 515 and sheath 540. The sheath 540 is placed over the stiffener 515 to form the stiffening element. According to one illustrative embodiment, the sheath 540 may be formed from PTFE or other polymer. For example, the sheath 540 may be formed from expanded PTFE. Expanded PTFE has high strength, chemical inertness, and a much lower modulus of elasticity than unexpanded PTFE. According to one illustrative embodiment, the expanded PTFE sheath or liner may be a tube with an inside diameter of 0.006 inches and an outside diameter of 0.007 inches. Additionally, the size and shape of the sheath 540 may be selected to allow more or less relative motion between the stiffening element 500 and the sheath and between the sheath and the flexible body 475. For example, in some embodiments, the sheath 540 may be sized to fit relatively loosely over the stiffening element 500. This will allow the stiffening element 500 to slide within the sheath 540 relatively easily. Alternatively, the sheath 540 may be sized to fit more tightly around the stiffening element 500. In this embodiment, there may be little or no relative motion of the stiffening element 500 within the sheath 540. Instead, the relative motion of the stiffening element 500 with respect to the flexible body 475, (Fig. 5) may take place primarily at the interface between the outer surface of the sheath 540 and the inner surface of the flexible body.

In some embodiments, the fit of the sheath 540 over the stiffening element may vary from location to location. For example, the sheath 540 may be formed so that it fits relatively loosely over the stiffening element 500 near its proximal and distal ends, but have a tighter fit in the center of the stiffening element. Additionally, the sheath 540 may cover the entire stiffening element 500 or only a portion of the stiffening element 500. For example, the sheath 540 may cover only the distal portion 515 of the stiffening element. Although the sheath 540 is shown with an open end and a closed end, the sheath may have both ends open or both ends closed.

Fig. 5B shows a cross-section of an illustrative stiffening element 500 with the sheath 540 in place over the stiffener 515. A cap 545 has been place over the distal end of the stiffening element. The cap 545 may be formed from the same or different material than the sheath 540. The cap 545 may be bonded to the tip of the stiffening element 500 or may be an integral part of the sheath 540.

Fig. 5C shows a cross-section of a distal portion of an illustrative cochlear lead. As shown in the figure, the stiffening element extends past the most distal electrode 465-1, with the cap 540 being located between the most distal electrode 465-1 and the tip 440 of the electrode array. The cap 545 may serve a number of purposes, including distributing the insertion forces over a larger area and preventing the stiffening element 500 from penetrating the flexible body 475.

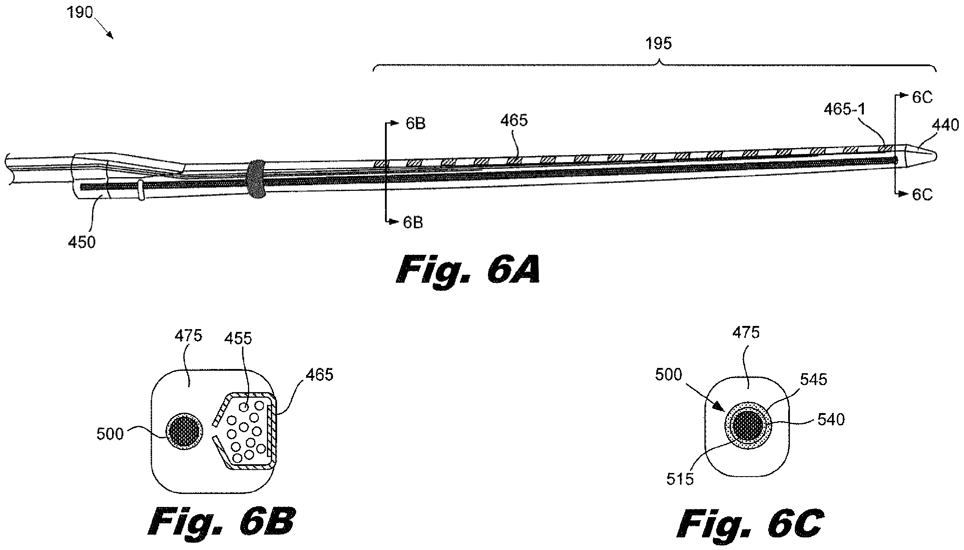

Fig. 6A is a partial side view of an illustrative cochlear lead 190. As discussed above, the cochlear lead 190 includes an electrode array 195 comprising electrodes 465, a lead body 445 carrying wires 455 that extend from the internal processor 185, Fig. 3 to the electrodes 465, a flexible body 475 on which electrodes 465 are disposed, a stiffening element 500 disposed within a portion of the flexible body 475, and a molded silicone rubber feature 450 proximal of the electrode array 195. The illustrative cochlear lead 190 further includes a cochleostomy marker 466 as a guide for positioning the electrode array 195. When the electrode array 195 is properly positioned within the cochlea, the cochleostomy marker 466 is positioned at or near the cochleostomy, and, the electrodes 465 are well positioned to stimulate the tonotopically-arranged groups of nerve endings.

The stiffening element 500 may be located within the flexible body 475 and extend from at or near the molded feature 450 to a location within the electrode array 195. According to one embodiment, the stiffening element 500 comprises or consists essentially of platinum. In other embodiments, it may be made of any metal material which provides the desired mechanical and chemical properties. These properties may include low yield strength and chemical inertness. According to one illustrative embodiment, the stiffening element 500 may comprise or consist of gold or a gold alloy.

According to one embodiment, stiffening element 500 extends approximately 2 mm to 6 mm from the cochleostomy. For example, the stiffening element may extend into the cochlea approximately 15 mm to 30 mm from the cochleostomy. In this illustrative embodiment, the stiffening element 500 extends from the molded rubber gripping feature 450 through the electrode array past the most distal electrode. The gripping feature 450 can be grasped by general or specialized surgical tools. The extension of the stiffening element into the gripping feature 450 allows these tools to grip the stiffening element through the encapsulation with minimal risk of damage to other components in the cochlear lead. The stiffening element can then be manipulated using the surgical tools to guide the cochlear lead into the desired position within the cochlea.

Fig. 6B shows a cross-section along line 6B-6B of a portion of the illustrative cochlear lead 190 in which the stiffening element 500 is disposed. The wires 455 may be shaped into a wire bundle by the electrode 465. Portions of the electrode, the wires, and the stiffening element 500 are encapsulated by the flexible body 475. In this particular embodiment, the electrodes 465 are disposed within the flexible body 475 on the medial wall of the electrode array. The stiffening element 500 is disposed in flexible body 475 opposite the electrodes 465.

Fig. 6C is a cross-sectional diagram along line 6C-6C taken through the distal end of the stiffening element 500. Because the distal end of the stiffening element 500 extends beyond the most distal electrode 465-1, only the cross section of the stiffening element 500 is shown within the flexible body 475. The cross sectional view shows the stiffener 515 surrounded by the sheath 540 and the cap 545.

The stiffness and ductility of the stiffening element 500 can be selected to significantly influence the amount of force required to insert the electrode array 195 into the cochlea. Alternatively or additionally, the geometry of the stiffening element can be altered along its length to create the desired mechanical properties. For example, the distal portion of the stiffener may have a variety of cross sectional geometries, including flattened, elliptical or circular. The cross sections may vary along the length of the stiffener. These cross sections can be selected to produce the desired stiffness, with lower stiffnesses typically desired near the distal end of the stiffener. The different cross sections may be formed in a variety of ways, including grinding, rolling, pressing, drawing, or other suitable technique. In some embodiments, the distal portion of the stiffener may have a number of micro-machined features that produce desired bending characteristics.

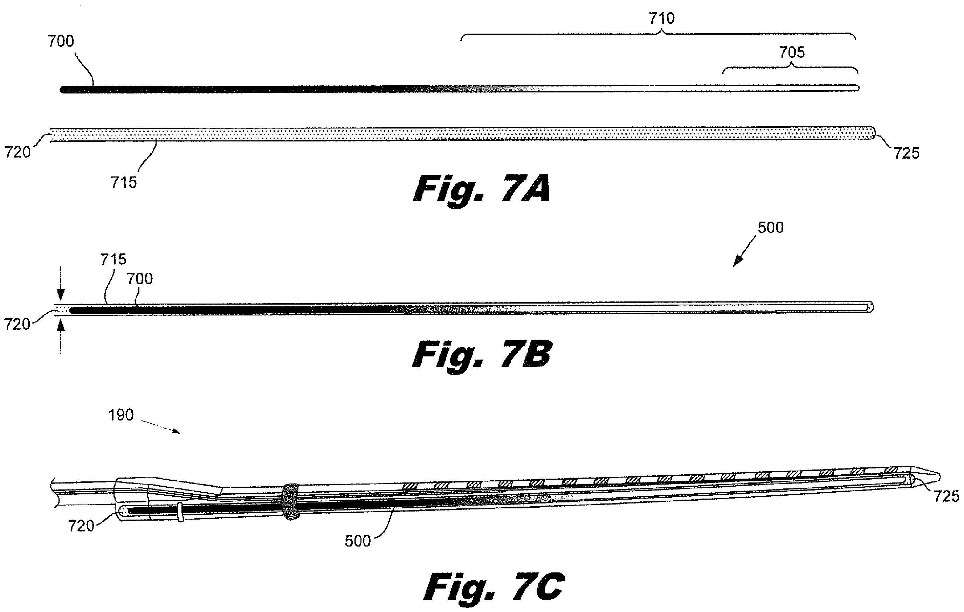

Figs. 7A-7C are cross sectional diagrams of steps in an illustrative process for making a cochlear lead with an integral stiffening element. In a first step, a stiffener 700 is shaped and annealed. The shaping process may include cutting the stiffener 700 to the desired length. In embodiments where the stiffening element is a metal, the distal portion 515 may be annealed. Annealing is a versatile heat treating process which alters the material properties such as yield strength and ductility of a metal. According to one illustrative example, the stiffener 700 may include platinum or a platinum alloy. In some embodiments, the stiffener 700 may consist essentially of platinum. Annealing may be used to produce a distal portion 710 having greater ductility and lower stiffness than the rest of the stiffener 700. This allows the distal portion to bend more easily to follow the curvature of the cochlear ducts. Further, annealing will allow the distal portion to maintain its bent shape after insertion into the cochlea. This can assist in holding the electrode array in place within the cochlea. For example, a distal portion 515 with high yield strength of 185 to 205 MPa may be more susceptible to migrate out from the cochlea due to strain energy. Conversely, a distal portion with a lower yield strength of 14 to 35 MPa may plastically conform to the shape of the cochlea and have a tendency to retain the electrode array in the cochlea.

A variety of other techniques, such as work hardening, could be used to modify the yield strength, malleability, ductility, stiffness, or other characteristics of the stiffener 700. According to one example, the distal portion 710 of the stiffener 700 is annealed and the tip portion 705 is annealed so that the metal is "dead soft." The term "dead soft" refers to the condition of maximum softness that is attainable in a metal or metal alloy through annealing. In other embodiments, the distal portion may be annealed but not to the extent that the metal is dead soft.

The annealing may be performed in a stepwise fashion, the entire tip portion 705 having a substantially uniform dead soft anneal and the remainder of the distal portion 710 being annealed to a lesser extent. The temper of the remainder of the stiffener 700 may remain in an "as drawn" state or may be heat treated to alter characteristics of the metal. In one embodiment, portions of the electrode that will be bent to follow the spiral of the cochlea are annealed and portions that remain in the relatively straight basal portion of the cochlea are more rigid. In alternative embodiments, the annealing may vary continuously along the length of the stiffener 700. According to one embodiment, the stiffener may be annealed from the distal tip up to approximately its midpoint.

The sheath 715 is formed by cutting it to length. In this example, the distal end 725 is closed while the proximal end 720 of the sheath is open. The sheath 715 is cut slightly longer than the stiffener. As discussed above, the sheath 715 may be sized to allow for motion of the stiffener 700 within it.

Fig. 7B shows the stiffener 700 inserted into the sheath 715. The proximal end 720 of the sheath is then fused or adhered shut. The stiffener is then entirely enclosed in the sheath to form the stiffening element 500. In some embodiments, the sheath may be substantially impermeable.

Fig. 7C is a side view of an illustrative cochlear lead 190 that includes the stiffening element 500, with the proximal end of the stiffening element 500 extending into the molded silicone rubber feature 450 and its distal end 725 extending to the tip 440 of the cochlear lead 190.

The sheath is only one illustrative example. In other examples, the stiffener 515 may be overcoated with a thin layer. This thin layer may be formed from a number of materials and applied in a variety of ways. In other embodiments, the flexible body may include a lumen may be lined with polytetrafluoroethylene PTFE or other friction reducing material. The stiffener can then be inserted into the lumen. In most cases, the stiffener can move relative to the flexible body 475 (Fig. 4) during bending. This reduces the overall bending stiffness of the cochlear lead 190 while still maintaining its resistance to kinking.

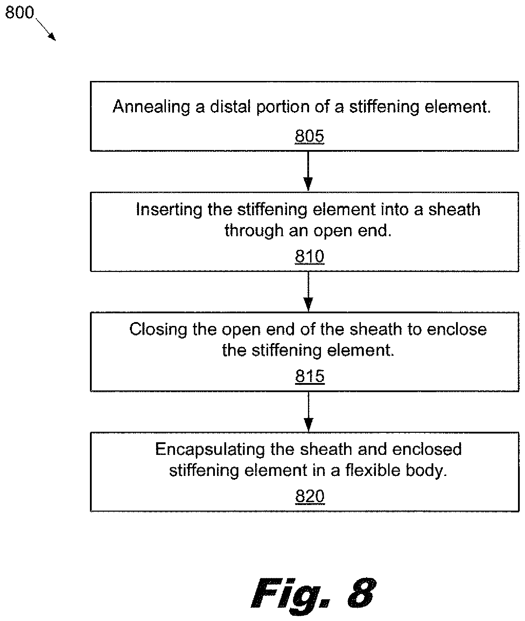

Fig. 8 is a flowchart showing an illustrative method for forming a cochlear lead. A stiffener is formed with the desired material, diameter, and length. For example, the stiffener may comprise or substantially consist of platinum. The distal portion of the stiffener is then annealed (step 805) so that the distal portion of the stiffener is substantially softer than a proximal portion. In some embodiments, the distal portion is approximately half of the total length the stiffener. In some implementations a tip portion of the stiffener may be annealed so that it is dead soft. The tip portion may be one quarter or less of the total length of the stiffener.

The sheath is then formed. As discussed above, the sheath may be a polymer tube that is selected to allow the stiffener to slide freely within its inner diameter. Additionally, the polymer material may be selected for its lubricity and biocompatibility. One end of the tube is closed, with the other end remaining open to receive the stiffener.

In some embodiments an additional cap is placed over the distal end of the sheath. The cap may be a flat plate or semi spherical shape that prevents puncture of the stiffening element through the flexible body. The cap may be used alone or in conjunction with the sheath. In other embodiment, the sheath is fused together to form a ball or caplike structure.

The stiffener is inserted into the sheath (step 810) and the ends of the sheath are closed (step 815). The sheath and the stiffener make up the stiffening element. The stiffening element is then encapsulated in the flexible body (step 820) with the electrodes and wires. A variety of techniques can be used to encapsulate the stiffening element in the flexible body. Illustrative examples of these techniques are described in WO 2011/149695 A1.

A variety of other steps can be taken to complete the manufacture of the cochlear lead. For example, these steps may include testing, sterilization, and packaging.

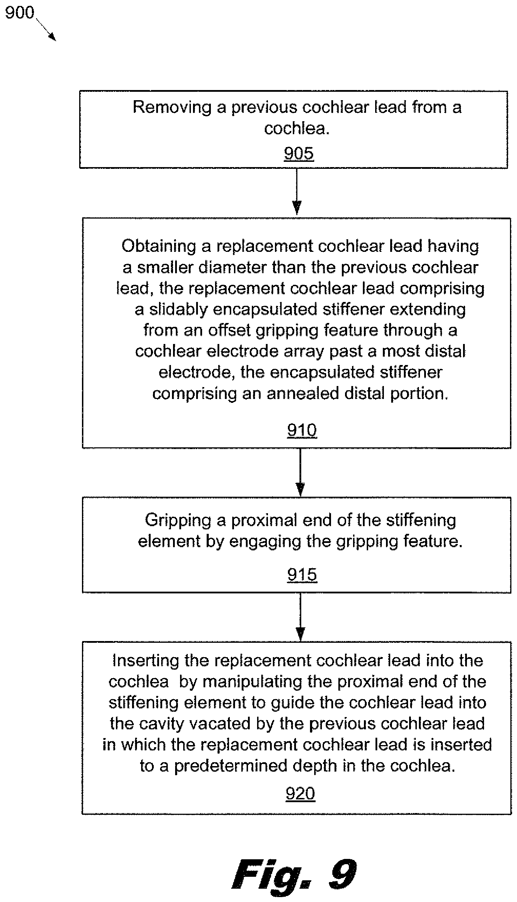

Fig. 9 is a flowchart showing an illustrative method for surgically implanting using a cochlear lead with a full length stiffening element during a revision surgery. The patient is prepared and the appropriate surgical openings made. The existing cochlear implant is removed from the patient, including removing a previous cochlear lead from the cochlea (step 905). The new cochlear implant, including a replacement cochlear lead with a full length slidably encapsulated stiffening element, is taken from its packaging (step 910). The replacement cochlear lead includes an offset gripping feature. The stiffening element extends from within the offset gripping feature through the electrode array and past the most distal electrode. In some instances, the new cochlear electrode array may have a smaller diameter than the cochlear electrode which was removed from the patient. This may allow for the new cochlear electrode to be more easily inserted into the opening that previously contained the old cochlear electrode.

The surgeon attaches the appropriate tool to the cochlear lead by gripping a proximal end of the stiffening element contained within the gripping feature (step 915). This tool may be a special purpose tool that is specifically adapted for insertion of the cochlear lead or may be a more general purpose surgical tool such as tweezers or forceps. In some cases, the surgeon may manually bend the distal portion of the cochlear lead to achieve the desired curvature. Because the distal portion of the cochlear lead is annealed, the cochlear lead will tend to maintain the curvature created by the surgeon. In other examples, the surgeon may leave the cochlear lead in its substantially straight configuration.

The surgeon then inserts the electrode array into the patient's cochlea by manipulating the proximal end of the stiffening element to guide the cochlear lead into the cavity vacated by the previous cochlear lead (step 920). The full length stiffening element provides the surgeon with increased control throughout the insertion process and allows slightly higher forces to be used without kinking or folding over the electrode array. The surgeon can use the increased control and resistance to kinking provided by the stiffening element to maneuver the cochlear lead past obstructions to a predetermined depth in the cochlea.

## Claims

1. A cochlear lead comprising:
a plurality of electrodes (465) configured to stimulate an auditory nerve from within a cochlea (150);
a flexible body (475) supporting the plurality of electrodes along a length of the flexible body; and
a stiffening element (500, 515, 700) made of a metal material, wherein the stiffening element is slidably encapsulated within the flexible body and is fully encapsulated within the cochlear lead (190) and wherein a distal portion of the stiffening element is configured to plastically deform upon insertion into a curved portion of the cochlea, **characterised in that** the stiffening element is extending past a most distal electrode (465-1).

2. The cochlear lead of claim 1, wherein the stiffening element (500, 515, 700) comprises a barrier element (540, 715) at least partially surrounding the stiffening element, and wherein at least portions of the stiffening element translate with respect to the flexible body (475) when the cochlear lead (190) bends.

3. The cochlear lead of claim 2, wherein the barrier element comprises a sheath (540, 715) that forms a physical barrier between exterior fluids and the stiffening element (500, 515, 700).

4. The cochlear lead of claim 3, wherein the sheath (540, 715) is a polymer coating adhered to the stiffening element (500), wherein the polymer coating and stiffening element slide with respect to the flexible body (475) when the cochlear lead (190) bends.

5. The cochlear lead of claim 3, wherein the sheath (540, 715) is closed on both ends and entirely surrounds the stiffening element (500, 515, 700).

6. The cochlear lead of claim 3, wherein the sheath (540, 715) is a polymer sock having an inner diameter sized such that the stiffening element (500, 515, 700) slides within the polymer sock.

7. The cochlear lead of claim 1, further comprising a gripping feature (450) extending from one side of the flexible body (475), wherein the stiffening element (500, 515, 700) extends into the gripping feature.

8. The cochlear lead of claim 1, further comprising a cap (545) on a distal end (725) of the stiffening element (500, 515, 700).

9. The cochlear lead of claim 8, wherein the cap (545) is disposed over a sheath (540, 715) surrounding the stiffening element (500, 515, 700).

10. The cochlear lead of claim 1, wherein the cochlear lead (190) is substantially straight and has a radius of curvature of no more than 20 mm when the cochlear lead is in its relaxed state.

11. The cochlear lead of claim 1, wherein the distal portion of the stiffening element (500, 515, 700) is dead soft.

12. The cochlear lead of claim 11, wherein the stiffening element (500, 515, 700) consists essentially of platinum.

13. The cochlear lead of claim 11, wherein the stiffening element (500, 515, 700) is annealed to approximately a midpoint of the stiffening element.

14. A method for forming a cochlear lead (190) comprising:
annealing a distal portion of a stiffening element (500, 515, 700);
inserting the stiffening element into a sheath (540, 715) through an open end;
closing the open end of the sheath to enclose the stiffening element; and
encapsulating the sheath and enclosed stiffening element in a flexible body (475) so as to fully encapsulate the stiffening element within the cochlear lead, wherein the stiffening element is positioned such within the flexible body that it extends past a most distal electrode (465-1) of a plurality of electrodes (465) configured to stimulate an auditory nerve from within a cochlea (150).

15. The method of claim 14, further comprising placing a cap (545) on a distal end of the stiffening element (500, 515, 700), wherein the sheath (540, 715) comprises polytetrafluoroethylene, and wherein closing the open end of the sheath comprises heating and compressing the open end of the sheath.

## Patentansprüche

1. Cochleazuleitung mit:
einer Mehrzahl von Elektroden (465) zum Stimulieren eines Hörnervs innerhalb einer Cochlea (150);
einem flexiblen Körper (475), der die Mehrzahl von Elektroden entlang einer Länge des flexiblen Körpers trägt; und
einem Versteifungselement (500, 515, 700), welches aus einem metallischen Material gefertigt ist, wobei das Versteifungselement gleitend innerhalb des flexiblen Körpers eingekapselt ist und vollständig innerhalb der Cochleazuleitung (190) eingekapselt ist, und wobei ein distaler Abschnitt des Versteifungselements ausgebildet ist, um sich beim Einführen in einen gekrümmten Abschnitt der Cochlea plastisch zu verformen, **dadurch gekennzeichnet, dass** sich das Versteifungselement über eine am weitesten distal gelegene Elektrode (465-1) hinaus erstreckt.

2. Cochleazuleitung gemäß Anspruch 1, wobei das Versteifungselement (500, 515, 700) ein Barrierenelement (540, 715) aufweist, welches das Versteifungselement mindestens teilweise umgibt, und wobei mindestens Abschnitte des Versteifungselements sich bezüglich des flexiblen Körpers (475) verschieben, wenn sich die Cochleazuleitung (190) biegt.

3. Cochleazuleitung gemäß Anspruch 2, wobei das Barrierenelement eine Hülse (540, 715) aufweist, welche eine physikalische Barriere zwischen externen Fluiden und dem Versteifungselement (500, 515, 700) bildet.

4. Cochleazuleitung gemäß Anspruch 3, wobei die Hülse (540, 715) eine Polymerbeschichtung ist, die an dem Versteifungselement (500) anhaftet, wobei die Polymerbeschichtung und das Versteifungselement bezüglich des flexiblen Körpers (475) gleiten, wenn sich die Cochleazuleitung (190) biegt.

5. Cochleazuleitung gemäß Anspruch 3, wobei die Hülse (540, 715) an beiden Enden geschlossen ist und das Versteifungselement (500, 515, 700) vollständig umgibt.

6. Cochleazuleitung gemäß Anspruch 3, wobei die Hülse (540, 715) ein Polymersack ist, der einen Innendurchmesser hat, der so gewählt ist, dass das Versteifungselement (500, 515, 700) innerhalb des Polymersacks gleitet.

7. Cochleazuleitung gemäß Anspruch 1, ferner versehen mit einem Griffmerkmal (450), welches sich von einer Seite des flexiblen Körpers (475) aus erstreckt, wobei sich das Versteifungselement (500, 550, 700) in das Griffmerkmal hinein erstreckt.

8. Cochleazuleitung gemäß Anspruch 1, ferner versehen mit einer Kappe (545) auf einem distalen Ende (725) des Versteifungselements (500, 515, 700).

9. Cochleazuleitung gemäß Anspruch 8, wobei die Kappe (545) über einer Hülse (540, 715) angeordnet ist, welche das Versteifungselement (500, 515, 700) umgibt.

10. Cochleazuleitung gemäß Anspruch 1, wobei die Cochleazuleitung (190) im Wesentlichen gerade ausgebildet ist und einen Krümmungsradius von nicht mehr als 20 mm aufweist, wenn sich die Cochleazuleitung in ihrem entspannten Zustand befindet.

11. Cochleazuleitung gemäß Anspruch 1, wobei der distale Abschnitt des Versteifungselements (500, 515, 700) maximal weich ist.

12. Cochleazuleitung gemäß Anspruch 11, wobei das Versteifungselement (500, 515, 700) im Wesentlichen aus Platin besteht.

13. Cochleazuleitung gemäß Anspruch 11, wobei das Versteifungselement (500, 515, 700) zu ungefähr einem Mittelpunkt des Versteifungselements wärmebehandelt ist.

14. Verfahren zum Bilden einer Cochleazuleitung (190), wobei:
ein distaler Abschnitt des Versteifungselements (500, 515, 700) wärmebehandelt wird;
das Versteifungselement durch ein offenes Ende in eine Hülse (540, 715) eingeführt wird; das offene Ende der Hülse geschlossen wird, um das Versteifungselement einzuschließen; und die Hülse und das eingeschlossene Versteifungselement in einem flexiblen Körper (475) eingekapselt werden, um das Versteifungselement innerhalb der Cochleazuleitung vollständig einzukapseln, wobei das Versteifungselement so in dem flexiblen Körper positioniert wird, dass es sich über eine am weitesten distal gelegene Elektrode (475-1) einer Mehrzahl von Elektroden (465) hinaus erstreckt, die zum Stimulieren eines Hörnervs innerhalb der Cochlea (51) vorgesehen sind.

15. Verfahren gemäß Anspruch 14, wobei ferner eine Kappe (545) auf einem distalen Ende des Versteifungselements (500, 515, 700) platziert wird, wobei die Hülse (540, 715) Polytetrafluorethylen aufweist, und wobei beim Schließen des offenen Endes der Hülse das offene Ende der Hülse erhitzt und komprimiert wird.

## Revendications

1. Conducteur cochléaire comprenant :
une pluralité d'électrodes (465) configurées pour stimuler un nerf auditif depuis l'intérieur d'une cochlée (150) ;
un corps souple (475) supportant la pluralité d'électrodes le long d'une longueur du corps souple ; et
un élément raidisseur (500, 515, 700) constitué d'un matériau métallique,
dans lequel l'élément raidisseur est encapsulé par glissement à l'intérieur du corps souple et est entièrement encapsulé à l'intérieur du conducteur cochléaire (190) et
dans lequel une partie distale de l'élément raidisseur est configurée pour se déformer plastiquement lors de l'insertion dans une partie incurvée de la cochlée,
**caractérisé en ce que** l'élément raidisseur s'étend au-delà d'une électrode la plus distale (465-1).

2. Conducteur cochléaire de la revendication 1, dans lequel l'élément raidisseur (500, 515, 700) comprend un élément faisant barrière (540, 715) entourant au moins partiellement l'élément raidisseur, et dans lequel au moins des parties de l'élément raidisseur se translatent par rapport au corps souple (475) quand le conducteur cochléaire (190) se plie.

3. Conducteur cochléaire de la revendication 2, dans lequel l'élément faisant barrière comprend une gaine (540, 715) qui forme une barrière physique entre des fluides extérieurs et l'élément raidisseur (500, 515, 700).

4. Conducteur cochléaire de la revendication 3, dans lequel la gaine (540, 715) est un revêtement polymère collé à l'élément raidisseur (500), le revêtement polymère et l'élément raidisseur glissant par rapport au corps souple (475) quand le conducteur cochléaire (190) se plie.

5. Conducteur cochléaire de la revendication 3, dans lequel la gaine (540, 715) est fermée aux deux extrémités et entoure entièrement l'élément raidisseur (500, 515, 700).

6. Conducteur cochléaire de la revendication 3, dans lequel la gaine (540, 715) est une chaussette polymère ayant un diamètre intérieur dimensionné de telle sorte que l'élément raidisseur (500, 515, 700) glisse à l'intérieur de la chaussette polymère.

7. Conducteur cochléaire de la revendication 1, comprenant en outre un élément de préhension (450) s'étendant depuis un côté du corps souple (475), l'élément raidisseur (500, 515, 700) s'étendant à l'intérieur de l'élément de préhension.

8. Conducteur cochléaire de la revendication 1, comprenant en outre un capuchon (545) sur une extrémité distale (725) de l'élément raidisseur (500, 515, 700).

9. Conducteur cochléaire de la revendication 8, dans lequel le capuchon (545) est disposé par-dessus une gaine (540, 715) entourant l'élément raidisseur (500, 515, 700).

10. Conducteur cochléaire de la revendication 1, le conducteur cochléaire (190) étant sensiblement rectiligne et ayant un rayon de courbure ne dépassant pas 20 mm quand le conducteur cochléaire est dans son état relâché.

11. Conducteur cochléaire de la revendication 1, dans lequel la partie distale de l'élément raidisseur (500, 515, 700) est extradouce.

12. Conducteur cochléaire de la revendication 11, dans lequel l'élément raidisseur (500, 515, 700) est essentiellement composé de platine.

13. Conducteur cochléaire de la revendication 11, dans lequel l'élément raidisseur (500, 515, 700) est recuit jusqu'à environ un milieu de l'élément raidisseur.

14. Procédé de formation d'un conducteur cochléaire (190) comprenant :
le recuit d'une partie distale d'un élément raidisseur (500, 515, 700) ;
l'insertion de l'élément raidisseur dans une gaine (540, 715) par une extrémité ouverte ;
la fermeture de l'extrémité ouverte de la gaine pour enfermer l'élément raidisseur ; et
l'encapsulation de la gaine et de l'élément raidisseur enfermé dans un corps souple (475) de manière à encapsuler entièrement l'élément raidisseur à l'intérieur du conducteur cochléaire, l'élément raidisseur étant positionné de telle sorte à l'intérieur du corps souple qu'il s'étend au-delà d'une électrode la plus distale (465-1) d'une pluralité d'électrodes (465) configurées pour stimuler un nerf auditif depuis l'intérieur d'une cochlée (150).

15. Procédé de la revendication 14, comprenant en outre la mise en place d'un capuchon (545) sur une extrémité distale de l'élément raidisseur (500, 515, 700), la gaine (540, 715) comprenant du polytétrafluoroéthylène, et la fermeture de l'extrémité ouverte de la gaine comprenant le chauffage et la compression de l'extrémité ouverte de la gaine.
